# EUROPÄISCHE PATENTSCHRIFT

(11) **EP 0 711 748 B1**
(45) Veröffentlichungstag und Bekanntmachung des Hinweises auf die Patenterteilung: **29.07.1998**
(21) Anmeldenummer: 95117460.6
(22) Anmeldetag: 06.11.1995
(51) Int. Cl.: C07C 67/347, C07C 69/716

(54) **Verfahren zur Herstellung von Formylcarbonsäureestern**
Process for the preparation of esters of formyl carboxylic acids
Procédé de fabrication d'esters d'acides formyle carboxyliques

(30) Priorität: 12.11.1994 DE 4440552
(43) Veröffentlichungstag der Anmeldung: 15.05.1996
(73) Patentinhaber: Celanese GmbH, 60439 Frankfurt (DE)
(72) Erfinder: Bahrmann, Helmut, Dr., D-46499 Hamminkeln (DE); Lappe, Peter, Dr., D-46599 Dinslaken (DE); Fell, Bernhard, Prof. Dr., D-52074 Aachen (DE); Leckel, Dieter, Dr., D-54292 Trier (DE); Schobben, Christian, D-52064 Aachen (DE)

(56) Entgegenhaltungen:
- EP-A- 0 157 316
- EP-A- 0 163 234
- PATENT ABSTRACTS OF JAPAN vol. 14 no. 294 (C-0732) ,26.Juni 1990 & JP-A-02 096549 (MITSUBISHI KASEI CORP) 9.April 1990,

## Beschreibung

Die Erfindung betrifft ein Verfahren zur Herstellung von Di- und Polyformylcarbonsäureestern durch Hydroformylierung von Estern mehrfach ungesättigter Fettsäuren in Gegenwart einer wäßrigen Lösung, die Rhodiumcarbonyl/Phosphin-Komplexverbindungen als Katalysator und weiterhin ein Tensid enthält.

Die Hydroformylierung ungesättigter Fettsäureester findet zunehmendes Interesse. Es beruht vor allem darauf, daß es sich bei den Einsatzmaterialien häufig um native Rohstoffe oder aus nativen Rohstoffen hergestellten Substanzen handelt, die in großen Mengen zur Verfügung stehen. Die Reaktionsprodukte der Hydroformylierung, Mono- oder Polyformylcarbonsäureester, die auch noch reaktive Doppelbindungen enthalten können, sind gesuchte Zwischenprodukte. Sie lassen sich zu vielfach genutzten Erzeugnissen wie Polyaminen, Polyurethanen, Alkydharzen, Weichmachern und synthetischen Schmierstoffen weiterverarbeiten.

Die Hydroformylierung höherer, mehrfach olefinisch ungesättigter Verbindungen ist bereits wiederholt untersucht worden. Problematisch ist im Falle dieser Reaktion das hohe Molekulargewicht von Ausgangsmaterial und Reaktionsprodukt, das eine Abtrennung und Rückführung des im Reaktionsprodukt homogen gelösten Katalysators, z.B. durch Destillation, unmöglich macht. Bei Einsatz mehrfach ungesättigter Verbindungen mit isolierten, aber nahe beieinanderliegenden Doppelbindungen gelingt die Hydroformylierung unter Vermeidung einer Doppelbindungsisomerisierung überdies nur mit Hilfe von Rhodiumcarbonyl/tert.-Phosphin-Komplexkatalysatorsystemen.

Ein für die Wirtschaftlichkeit des Verfahrens entscheidendes Problem ist die verlustfreie Abtrennung des homogen gelösten Katalysatorsystems von den Reaktionsprodukten und seine Rückführung in aktiver Form in den Hydroformylierungsreaktor. Bisher ist es lediglich gelungen, den Rhodium/Phosphin-Katalysator aus den Formylfettsäureester enthaltenden Reaktionsgemischen der Hydroformylierung einfach ungesättigter Fettsäureester abzutrennen. Die Arbeitsweise erfordert jedoch aufwendige Maßnahmen, überdies fällt der Katalysator in inaktiver Form an und der Phosphinanteil des Katalysatorsystems geht vollständig verloren (J.Amer. Oil Chem. Soc. Vol. 50, 455 (1973)).

Linol- und Linolensäuremethylester können in Gegenwart heterogenisierter Rhodiumcarbonyl/Phosphin-Komplexkatalysatoren auf Polysiloxanbasis hydroformyliert werden (Chemiker-Zeitung, 115 (1991, S. 39 ff)). Das Verfahren liefert bei Einsatz von Linolsäuremethylester Mono- und Diformylstearylsäureester in Ausbeuten bis zu 95 %, bezogen auf den eingesetzten zweifach ungesättigten Ester. Auch Linolensäure ergibt bei der Hydroformylierung in Gegenwart des genannten Katalysatorsystems lediglich die Diformylverbindung, Triformylprodukte werden dagegen allenfalls in untergeordneten Mengen erhalten. Der Rhodiumaustrag liegt im Mittel bei etwa 0,5 % des ursprünglich eingesetzten Edelmetalls. Nicht auszuschließen ist, daß ein Anteil des Katalysatormetalls im Gleichgewicht mit dem fixierten Metall homogen gelöst vorliegt, so daß die Hydroformylierung nicht nur am Festbettkatalysator, sondern auch in Lösung stattfindet.

Es bestand daher die Aufgabe, ein Verfahren zu entwickeln, das es erlaubt, Ester ungesättigter Fettsäuren zu hydroformylieren, wobei mehrfach ungesättigte Fettsäureester nicht nur partiell, sondern vollständig hydroformyliert werden. Darüber hinaus sollen Edelmetallverluste weitgehend vermieden werden.

Die vorstehend beschriebene Aufgabe wird gelöst durch ein Verfahren zur Herstellung von Di- und Polyformylcarbonsäureestern. Es ist dadurch gekennzeichnet, daß man Ester aus mehrfach ungesättigter Fettsäuren und niedermolekularen Monoalkoholen bei 100 bis 180°C und 5 bis 35 MPa in Gegenwart einer wäßrigen Lösung, die Rhodium-Phosphin-Komplexverbindungen als Katalysatoren und weiterhin ein Tensid enthält, mit Kohlenmonoxid und Wasserstoff umsetzt.

Die Hydroformylierung von Olefinen mit mehr als 6 Kohlenstoffatomen im Molekül in Gegenwart einer wäßrigen Lösung, die als Katalysator Rhodium-Komplexverbindungen und überdies als Lösungsvermittler ein quartäres Ammoniumsalz enthält, ist aus der EP-B-157 316 bekannt. Eine Weiterentwicklung dieses Prozesses ist Gegenstand der EP-B-163 234. Nach der Lehre dieser Patentschrift werden C₆- bis C₂₀-Olefine mit Wasserstoff und Kohlenmonoxid in Gegenwart von Rhodium oder einem sulfonierten Arylphosphin umgesetzt, dessen Kation ein quartäres Ammoniumion ist.

Beide Verfahren betreffen ausschließlich die Umsetzung von einfach ungesättigten Verbindungen, die darüber hinaus keine funktionellen Gruppen enthalten. Überraschenderweise gelingt es nach dem neuen Prozeß, mehrere im Estermolekül enthaltene und auch innenständige Doppelbindungen gleichzeitig zu hydroformylieren, so daß z.B. aus zweifach ungesättigten Verbindungen Diformylprodukte und aus dreifach ungesättigten Verbindungen Triformylprodukte erhalten werden.

Ausgangsverbindungen für das erfindungsgemäße Verfahren sind Ester, deren eine Komponente mehrfach, insbesondere zweifach und dreifach ungesättigte Fettsäuren mit 8 bis 25, vorzugsweise 10 bis 20 Kohlenstoffatomen im Molekül und deren andere Komponente gesättigte Monoalkohole mit 1 bis 10 Kohlenstoffatomen im Molekül, vorzugsweise Methanol ist. Man erhält diese Ester aus natürlichen Ölen, die gegebenfalls zuvor raffiniert und destilliert wurden, durch Umesterung. Beispiele für natürliche Öle als Basis für die Säurekomponente der Ausgangsester sind Baumwollsaatöl, Distelöl, Erdnußöl, Kürbiskernöl, Leinöl, Maiskeimöl, Sojaöl und Sonnenblumenöl.

Als Katalysatoren werden im beanspruchten Verfahren Rhodiumverbindungen eingesetzt, die in komplexer Bindung wasserlösliche Phosphine enthalten, d.h. Salze, deren Anion ein Phosphin ist, das mindestens einen sulfonierten oder carboxylierten aromatischen Rest enthält. Der Begriff Phosphin schließt auch solche Verbindungen des dreiwertigen Phosphors ein, in denen das Phosphoratom Bestandteil eines heterocyclischen Ringes ist. Der aromatische Rest kann unmittelbar oder über andere Gruppen an das Phosphoratom des Phosphins gebunden sein. Beispiele für aromatische Reste sind der Phenyl- und der Naphthylrest. Sie können einfach und mehrfach sulfoniert oder carboxyliert und darüber hinaus durch weitere Atomgruppierungen oder Atome wie Alkyl, Hydroxyl, Halogenid substituiert sein. Monophosphinanionen leiten sich bevorzugt von Verbindungen der allgemeinen Formel (1) 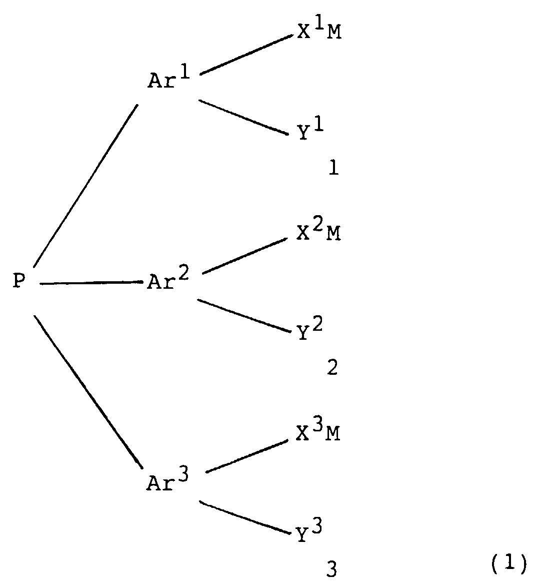 ab. Hierbei bedeuten Ar¹, Ar², Ar³ jeweils eine Phenyl- oder Naphthylgruppe, Y¹, Y², Y³ jeweils eine geradkettige oder verzweigte Alkylgruppe mit 1 bis 4 C-Atomen, eine Alkoxygruppe, ein Halogenatom, die OH-, CN-, NO₂- oder R¹R²-N-Gruppen, in der R¹ und R² für jeweils eine geradkettige oder verzweigte Alkylgruppe mit 1 bis 4 C-Atomen stehen; X¹, X², X³ ist jeweils ein Sulfonat-(SO₃ ⁻-) und/oder Carboxylat-(COO⁻-) Rest, n₁, n₂, n₃ sind gleiche oder verschiedene ganze Zahlen von 0 bis 5, M ist ein Alkalimetallion, ein chemisches Äquivalent eines Erdalkalimetall- oder Zinkions, ein Ammonium oder quaternäres Ammoniumion der allgemeinen Formel N(R³R⁴R⁵R⁶)⁺ in der R³, R⁴, R⁵, R⁶ jeweils für eine geradkettige oder verzweigte Alkylgruppe mit 1 bis 4 C-Atomen steht.

Bevorzugt sind Verbindungen der vorstehend beschriebenen allgemeinen Formel, in der Ar¹, Ar², Ar³ jeweils einen Phenylrest und X¹, X², X³ jeweils einen in meta-Stellung zum Phosphor befindlichen Sulfonatrest bedeuteten (Tris(m-sulfonatophenyl)phosphin, abgekürzt TPPTS).

Eine weitere Gruppe als Katalysatorkomponente geeigneter Monophosphine erhält man durch Sulfalkylierung von Dialkyl- oder Diarylphosphinen mit 1,2-, 1,3- oder 1,4-Sultonen 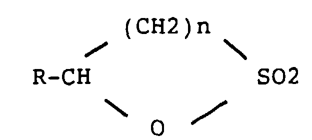 (mit n = 0, 1 oder 2 und R = H, Alkyl),
z.B. entsprechend 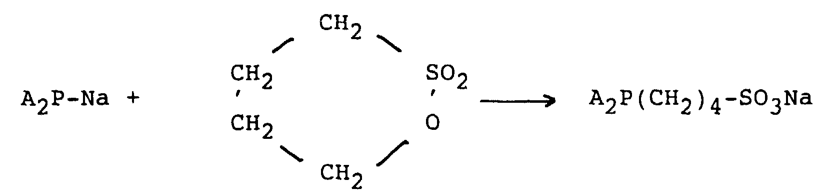 wobei A für gleiche oder verschiedene Alkyl- oder Arylreste steht.

Das Anion kann außer von Monophosphinen auch von Polyphosphinen, insbesondere Diphosphinen, die mindestens einen sulfonierten oder carboxylierten aromatischen Rest enthalten, gebildet werden. Diphosphinanionen leiten sich bevorzugt von Diarylverbindungen der allgemeinen Formel (2) 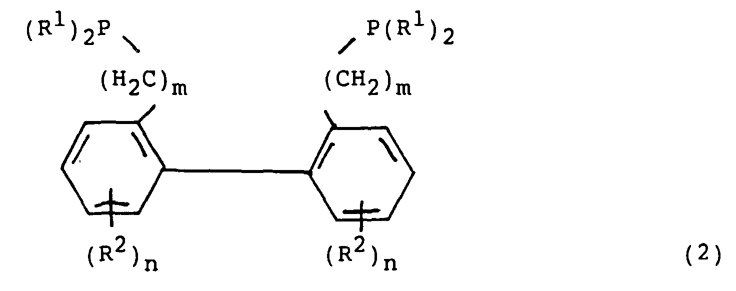 ab, die durch mindestens einen Sulfonat-(SO₃ ⁻)-Rest oder Carboxylat-(COO⁻)Rest substituiert sind. In der allgemeinen Formel stehen R¹ für gleiche oder verschiedene Alkyl-, Cycloalkyl-, Phenyl-, Tolyl- oder Naphthylreste, R² ist gleich oder verschieden und bedeutet Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen annelierten Benzolring, m ist gleich oder verschieden und steht für ganze Zahlen von 0 bis 5 und n ist ebenfalls gleich oder verschieden und steht für ganze Zahlen von 0 bis 4. Bevorzugt werden die sulfonierten Verbindungen, die nach gängigen Methoden zugänglich sind. Bewährte Vertreter dieser Verbindungsklasse sind die durch Sulfonierung von 2,2'-Bis(diphenylphosphinomethyl)-1,1'-biphenyl oder 2,2'-Bis(diphenylphosphinomethyl)-1,1'-binaphthyl erhaltenen Produkte. Als Beispiel des Anions einer heterocyclischen Phosphorverbindung ist das 3,4-Dimethyl-2,5,6-tris(p-sulfonatophenyl)-1-phosphanorbornadien zu nennen.

Üblicherweise verwendet man als Katalysatorbestandteil die Alkalisalze oder die Ammoniumsalze, insbesondere die Natriumsalze der sulfonierten oder carboxylierten Phosphine.

Ein wesentliches Merkmal der neuen Arbeitsweise ist der Zusatz eines Tensids (auch als Lösungsvermittler, Phasentransfer, oberflächenaktives oder amphiphiles Reagens bezeichnet) zu der wäßrigen Katalysatorlösung. Unter Tensiden versteht man Stoffe oder Stoffgemische, die sowohl mit der wäßrigen Phase (dem Katalysator) als auch mit der organischen Phase (dem ungesättigten Fettsäureester) verträglich und in beiden Phasen zumindest bei erhöhten Temperaturen löslich sind. Aufgabe der Tenside ist die Verbesserung der Löslichkeit der Fettsäureester in der Katalysatorlösung. Sie erfolgt durch Aggregation der Tensidteilchen zu Mizellen oberhalb der für jedes Tensid charakteristischen kritischen Mizellbildungskonzentration (c.m.c., vgl. Ullmanns Encyclopädie der technischen Chemie 4. Auflage, 1982, Band 22, Seiten 464, 465). In die Mizellen lagern sich die Estermoleküle ein und werden in dieser Form in die wäßrige Katalysatorphase transportiert, in der die Umsetzung mit dem Synthesegas erfolgt.

Entsprechend ihrem chemischen Aufbau unterscheidet man anionische Tenside wie die Seifen, Alkylsulfate, Alkylbenzolsulfonate und -phosphate, kationische Tenside, deren wichtigste Vertreter die Tetraalkylammoniumsalze sind, amphotere Tenside (Amphotenside) die zwitterionische hydrophile Gruppen aufweisen und für die Aminocarbonsäuren, Betaine, Sulfobetaine sowie Aminoxide beispielhaft sind und schließlich nichtionische Tenside, zu denen Alkyl- und Alkylphenylpolyethylenglykoläther, Fettsäurealkylolamide und Saccharosefettsäureester zählen.

Bevorzugt werden zur Anwendung im erfindungsgemäßen Verfahren Amphotenside und insbesondere Kationentenside, z.B. Tetrahexylammoniumbromid, Tetradecylammoniumbromid, N-Dodecyl-N,N,N-trimethylammoniumbromid, N-Tetradecyl-N,N,N-trimethylammoniumbromid, N-Hexadecyl-N,N,N-trimethylammoniumbromid, N-Octadecyl-N,N,N-trimethylammoniumbromid und Amphotenside, wie N,N-Dimethyldodecylammoniumbetain, N,N-Dimethyloctylamin-N-oxid, N,N,Dimethyldecylamin-N-oxid, N,N-Dimethyldodecylamin-N-oxid, N,N-Dimethyltetradecylamin-N-oxid. Die Tenside können als einheitliche Substanzen oder auch als Gemische eingesetzt werden. Die Konzentration des Tensids in der wäßrigen Katalysatorlösung liegt oberhalb der kritischen Mizellbildungskonzentration, die unter den Reaktionsbedingungen der Hydroformylierungsreaktion gegeben ist.

Die Umsetzung der mehrfach ungesättigten Fettsäureester mit Wasserstoff und Kohlenmonoxid erfolgt bei Temperaturen von 100 bis 180°C, insbesondere 120 bis 140°C und Drücken von 5 bis 35 MPa, vorzugsweise 15 bis 20 MPa.

Der Katalysator kann dem Reaktionssystem präformiert zugesetzt werden. Mit gleich gutem Erfolg läßt er sich aber auch aus den Komponenten Rhodium oder Rhodiumverbindung und der wäßrigen Lösung des sulfonierten oder carboxylierten Phosphins unter Reaktionsbedingungen im Reaktionsgemisch, also in Gegenwart des Fettsäureester, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wäßrigen Katalysatorlösung beträgt 100 bis 600 Gew.-ppm, vorzugsweise 300 bis 400 Gew.-ppm, bezogen auf die Lösung. Um eine Isomerisierung der ungesättigten Fettsäureester auszuschließen, wird das sulfonierte bzw. carboxylierte Phosphin in einer solchen Menge eingesetzt, daß je mol Rhodium mindestens 20 mol, vorzugsweise 40 bis 80 mol, P(III) vorliegen.

Der pH-Wert der wäßrigen Katalysatorlösung soll einen Wert von 3 nicht unterschreiten. Allgemein stellt man einen pH-Wert von 5 bis 10, vorzugsweise 6 bis 8, ein.

Die Zusammensetzung des Synthesegases, d.h. das Verhältnis von Kohlenmonoxid zu Wasserstoff, kann in weiten Grenzen variiert werden. Im allgemeinen setzt man Synthesegas ein, in dem das Volumenverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das erfindungsgemäße Verfahren wird durch die nachfolgenden Beispiele erläutert, jedoch nicht auf die dargestellten Ausführungsformen beschränkt.

### Versuchsdurchführung

Zur Synthese der Di- und Triformylderivate von Fettsäuremethylestern durch Hydroformylierung mehrfach ungesättigter Fettsäuremethylester wurde entweder technisches Leinölfettsäuremethylestergemisch der Zusammensetzung 55 % Linolensäuremethylester, 15 % Linolsäuremethylester und 20 % Ölsäuremethylester, Rest gesättigte Fettsäuremethylester oder ein aus 90 % Linolen- und 10 % Linolsäuremethylester bestehendes Gemisch eingesetzt. Die Umsetzungen erfolgten in 160 ml-V4A-Stahlautoklaven mit druckfesten Tropftrichter, Druckaufnehmer, Berstscheibe und Thermoelement. Ein magnetgekoppeltes Propellerrührwerk mit Begasungsbohrungen sorgt für die intensive Durchmischung des Reaktionssatzes.

Die Herstellung der Katalysatorlösung erfolgte in einem mit Argon gespülten Schlenkrohr, in dem die berechneten Mengen Rhodiumverbindung (Rh₄(CO)₁₂, HRh(CO)(NaTPPTS)₃ oder Rh₂(SO₄)₃) Phosphinligand, sauerstofffreies Wasser und Tensid eingefüllt wurde. Der pH-Wert der Katalysatorlösung wurde mit NaHCO₃ oder Alkalihydroxid eingestellt.

Zur Herstellung des eigentlichen Hydroformylierungskatalysators wurde die Katalysatorlösung in einem mit Argon begasten und mit Synthesegas gespülten Autoklaven unter Rühren und unter den Temperatur- und Druckbedingungen der Hydroformylierung 1 h mit Synthesegas vorbehandelt. Anschließend wurde der ungesättigte Ester zugetropft.

Die Druckabnahme konnte während der Umsetzung mit einem Druckaufnehmer mit angeschlossenem Schreiber verfolgt werden. Nach beendeter Reaktion wurde der Autoklav abgekühlt, langsam entspannt und das Reaktionsgemisch in einen Scheidetrichter überführt. Wäßrige und organische Phase wurden getrennt, die organische Phase wurde in Ether aufgenommen und zweimal mit der doppelten Menge destilliertem Wasser gewaschen. Bei vermutetem geringem Rhodiumaustrag in die organische Phase wurde zusätzlich mit NaTPPTS-Lösung nachgewaschen. Die organische Phase wurde über Na₂SO₄ getrocknet, filtriert, der Ether abdestilliert und das Hydroformylierungsprodukt analysiert.

Die Hydrierung des Hydroformylierungsproduktes zu den entsprechenden Hydroxymethylverbindungen erfolgte mit 10 Gew.-% (bezogen auf das Hydroformylierungsprodukt) in Methanol aufgeschlämmtem Raney-Nickel als Katalysator bei 100°C und 14 MPa H₂-Druck. Als Lösungsmittel wurde die gleiche Volumenmenge Methanol eingesetzt. Das durch Filtration vom Katalysator und durch Destillation vom Methanol befreite Reaktionsprodukt wurde durch seine fettchemischen Kenndaten (Jodzahl, Carbonylzahl, Hydroxylzahl) charakterisiert.

### Beispiel 1

In einem 160 ml-Magnetrührautoklav wurden 10 g technisches Leinölfettsäureestergemisch (Estergemisch bezogen auf die Katalysatorphase) zusammen mit 20 cm³ einer wäßrigen Katalysatorlösung vorgelegt, die 0.08 mmol (200 Gew.-ppm) Rh, 1.6 mmol NaTPPTS (P/Rh-Verhältnis 20), 3.2 mmol Tetradecyl-trimethylammoniumbromid (7.5-fache c.m.c.) enthielt und mit Na₂CO₃ auf einen pH-Wert von 8 eingestellt worden war. Bei einem Reaktionsdruck von 20 MPa und einer Temperatur von 120°C wurden die beiden Flüssigphasen und die Gasphase intensiv gemischt. Nach einer Reaktionszeit von 12 Stunden wurde der Autoklav abgekühlt, entspannt und das Reaktionsprodukt, wie oben beschrieben, aufgearbeitet. Der Umsatz betrug 100 %. Das Reaktionsprodukt hatte eine Zusammensetzung von 26 Gew.-% Monoformylprodukt, bezogen auf das eingesetzte Estergemisch, 30 Gew.-% Diformylprodukt, bezogen auf die Linol- und Linolensäuremethylesteranteile im Estergemisch und 47 Gew.-% Trishydroformylierungsprodukte, bezogen nur auf den Linolensäuremethylesteranteil im Estergemisch. Die vorstehende Berechnungsweise der Ausbeute wurde auch bei allen anderen Versuchen mit dem technischen Leinölfettsäuremethylestergemisch eingehalten.

### Beispiele 2 - 6

Technisches Leinölfettsäuremethylestergemisch wurde, wie in Beispiel 1 beschrieben, jedoch unter Variation des Tensids in Gegenwart des wasserlöslichen Rhodiumcarbonyl/NaTPPTS-Katalysatorsystems hydroformyliert. Die Ergebnisse sind in Tabelle 1 zusammengefaßt.

**Tabelle 1**

| | Tensid | pH-Wert Kat.-Lsg. | MF | DF | TF | Umsatz |
|---|---|---|---|---|---|---|
| | | | [ Gew.-% ] | | | |
| Beisp. 2 | [C₁₆H₃₃N⁺(CH₃)₃]Br | 8 | 30 | 33 | 42 | 94 |
| Beisp. 3 | [C₁₈H₃₇N⁺(CH₃)₃]Br | 8 | 29 | 44 | 40 | 100 |
| Beisp. 4 | [C₁₂H₂₅N⁺(CH₃)₃]Br | 8 | 33 | 26 | 29 | 96 |
| Beisp. 5 | C₁₄H₂₉(CH₃)₂N - O | 5.6 | 32 | 41 | 38 | 99 |
| Beisp. 6 | [C₁₂H₂₅(CH₃)₂N⁺]CH₂COO- | 5.6 | 39 | 17 | 5 | 78 |
| (MF, DF, TF = Mono-, Di-, Triformylprodukte) | | | | | | |

### Beispiel 7

Technisches Leinölfettsäureestergemisch wurde, wie in Beispiel 1 beschrieben, jedoch bei dreifacher Rhodiumkonzentration (600 ppm Rhodium) und doppeltem P/Rh-Verhältnis (40 : 1) hydroformyliert. Der Umsatz war quantitativ. Das Produktgemisch enthielt:
27 Gew.-% Monoformylprodukte
30 Gew.-% Diformylprodukte
52 Gew.-% Triformylprodukte

### Beispiele 8 - 10

Entscheidenden Einfluß auf die mizellare Zweiphasenhydroformylierung hat die Tensidkonzentration, wie die folgenden Beispiele 8 - 10 zeigen. Damit eine mizellar gestützte Hydroformylierung ablaufen kann, muß als Mindestkonzentration die kritische Mizellbildungskonzentration c.m.c. des Tensids eingehalten werden. Im prämizellaren Bereich (0.85-fache c.m.c.) ist zwar bereits eine Hydroformylierung möglich, siehe Beispiel 8, aber mit steigender Tensidkonzentration nimmt die Reaktivität stark zu. Die Ausbeuten, vor allem an Triformylprodukten, steigen an und der Umsatz wird quantitativ.

Die Ergebnisse von mehreren Versuchen mit unterschiedlichen Konzentrationen des Kationtensids Tetradecyltrimethylammoniumbromid sind in der Tabelle 2 zusammengestellt. Die Reaktionsbedingungen entsprechen denen des Beispiels 1, jedoch betrug die Rh-Konzentraion in der wäßrigen Phase 275 Gew.-ppm. Das Volumenverhältnis von Katalysatorphase zu organischer Phase war 3 : 1.

**Tabelle 2**

| | Tensidkonz. [mol.l-1] | x-fache c.m.c. | Umsatz [Gew.-%] | MF | DF | TF |
|---|---|---|---|---|---|---|
| | | | | [ Gew.-% ] | | |
| Beisp. 8 | 0.018 | 0.85 | 55 | 32 | 19 | 11 |
| Beisp. 9 | 0.053 | 2.50 | 95 | 29 | 24 | 33 |
| Beisp. 10 | 0.107 | 5.00 | 100 | 27 | 33 | 52 |

## Patentansprüche

1. Verfahren zur Herstellung von Di- und Polyformylcarbonsäureestern, dadurch gekennzeichnet, daß man Ester aus mehrfach ungesättigten Fettsäuren und niedermolekularen Monoalkohlen bei 100 bis 180°C und 5 bis 35 MPa in Gegenwart einer wäßrigen Lösung, die Rhodium-Phosphin-Komplexverbindungen als Katalysatoren und weiterhin einen Lösungsvermittler (Tensid) enthält, mit Kohlenmonoxid und Wasserstoff umsetzt.

2. Verfahren nach Anspruch 1, dadurch gekennzeichnet, daß die Fettsäuren zwei- oder dreifach ungesättigt sind und 8 bis 25 Kohlenstoffatome im Molekül enthalten.

3. Verfahren nach Anspruch 1 oder 2 dadurch gekennzeichnet, daß die ungesättigten Fettsäuren 10 bis 20 Kohlenstoffe im Molekül enthalten.

4. Verfahren nach einem oder mehreren der Ansprüche 1 bis 3, dadurch gekennzeichnet, daß die niedermolekularen Monoalkohole gesättigt sind und 1 bis 10 Kohlenstoffe im Molekül enthalten.

5. Verfahren nach einem oder mehreren der Ansprüche 1 bis 4, dadurch gekennzeichnet, daß der Monoalkohol Methanol ist.

6. Verfahren nach einem oder mehreren der Ansprüche 1 bis 5, dadurch gekennzeichnet, daß das Phosphin ein Monophosphin der allgemeinen Formel 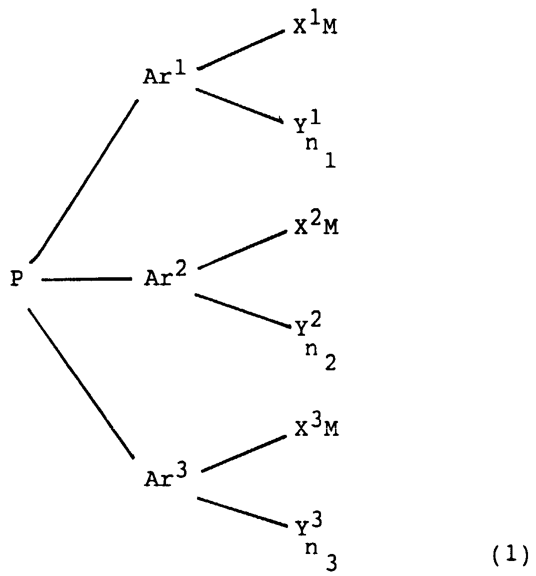 ist, in der Ar¹, Ar², Ar³ jeweils eine Phenyl- oder Naphthylgruppe bedeuten, Y¹, Y², Y³ jeweils eine geradkettige oder verzweigte Alkylgruppe mit 1 bis 4 C-Atomen, eine Alkoxygruppe, ein Halogenatom, die OH-, CN-, NO₂- oder R¹R²-N-Gruppen, in der R¹ und R² für jeweils eine geradkettige oder verzweigte Alkylgruppe mit 1 bis 4 C-Atomen stehen; X¹, X², X³ ist jeweils ein Sulfonat-(SO₃ ⁻-) und/oder Carboxylat-(COO⁻-) Rest, n₁, n₂, n₃ sind gleiche oder verschiedene ganze Zahlen von 0 bis 5, M ist ein Alkalimetallion, ein chemisches Äquivalent eines Erdalkalimetall- oder Zinkions, ein Ammonium oder quaternäres Ammoniumion der allgemeinen Formel N(R³R⁴R⁵R⁶)⁺ in der R³, R⁴, R⁵, R⁶ jeweils für eine geradkettige oder verzweigte Alkylgruppe mit 1 bis 4 C-Atomen steht.

7. Verfahren nach einem oder mehreren der Ansprüche 1 bis 5, dadurch gekennzeichnet, daß man als Monophosphine die Umsetzungsprodukte von Dialkyl- oder Diarylphosphinen mit 1,2-, 1,3- oder 1,4-Butansultonen einsetzt.

8. Verfahren nach einem oder mehreren der Patentansprüche 1 bis 5, dadurch gekennzeichnet, daß als Phosphin Diphosphine der allgemeinen Formel 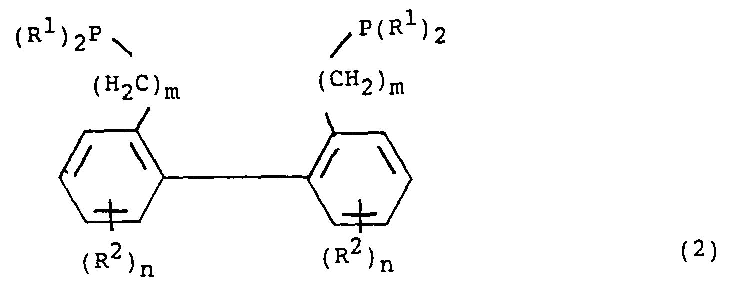 eingesetzt werden, in der R¹ für gleiche oder verschiedene Alkyl-, Cycloalkyl-, Phenyl, Tolyl- oder Naphthylreste stehen, R² gleich oder verschieden ist und Wasserstoff, Alkyl- oder Alkoxyreste mit 1 bis 14 Kohlenstoffatomen, Cycloalkyl-, Aryl- oder Aroxyreste mit 6 bis 14 Kohlenstoffatomen oder einen annelierten Benzolring bedeutet, m gleich oder verschieden ist und für ganze Zahlen von 0 bis 5 und n ebenfalls gleich oder verschieden ist und für ganze Zahlen von 0 bis 4 stehen.

9. Verfahren nach einem oder mehreren der Ansprüche 1 bis 8, dadurch gekennzeichnet, daß als Tenside Kationentenside oder Amphotenside eingesetzt werden.

10. Verfahren nach Anspruch 9, dadurch gekennzeichnet, daß die Kationentenside Tetraalkylammoniumsalze sind.

11. Verfahren nach Anspruch 9, dadurch gekennzeichnet, daß die Amphotenside Betaine oder Aminoxide sind.

12. Verfahren nach einem oder mehreren der Ansprüche 1 bis 11, dadurch gekennzeichnet, daß die Konzentration des Tensids in der wäßrigen Katalysatorlosung oberhalb der kritischen Mizellbildungskonzentration liegt.

13. Verfahren nach einem oder mehreren der Ansprüche 1 bis 12, dadurch gekennzeichnet, daß die Umsetzung bei 120 bis 140°C und 15 bis 20 MPa erfolgt.

14. Verfahren nach einem oder mehreren der Ansprüche 1 bis 13, dadurch gekennzeichnet, daß die Rhodiumkonzentration in der wäßrigen Katalysatorlösung 100 bis 600 Gew.-ppm, vorzugsweise 300 bis 400 Gew.-ppm, bezogen auf die Lösung beträgt.

15. Verfahren nach einem oder mehreren der Ansprüche 1 bis 14, dadurch gekennzeichnet, daß je mol Rhodium mindestens 20 mol, vorzugsweise 40 bis 80 mol P(III) in Form eines Phosphins vorliegen.

16. Verfahren nach einem oder mehreren der Ansprüche 1 bis 15, dadurch gekennzeichnet, daß der pH-Wert der wäßrigen Katalysatorlösung mindestens 3, vorzugsweise 5 bis 10 und insbesondere 6 bis 8 ist.

## Claims

1. A process for preparing di- and polyformylcarboxylic esters, which comprises reacting esters of multiply unsaturated fatty acids and low molecular weight monoalcohols with carbon monoxide and hydrogen at from 100 to 180°C and from 5 to 35 MPa in the presence of an aqueous solution containing rhodium-phosphine complexes as catalysts and additionally a solubilizer (surfactant).

2. The process as claimed in claim 1, wherein the fatty acids are doubly or triply unsaturated and contain from 8 to 25 carbon atoms in the molecule.

3. The process as claimed in claim 1 or 2, wherein the unsaturated fatty acids contain from 10 to 20 carbon atoms in the molecule.

4. The process as claimed in one or more of claims 1 to 3, wherein the low molecular weight monoalcohols are saturated and contain from 1 to 10 carbon atoms in the molecule.

5. The process as claimed in one or more of claims 1 to 4, wherein the monoalcohol is methanol.

6. The process as claimed in one or more of claims 1 to 5, wherein the phosphine is a monophosphine of the formula 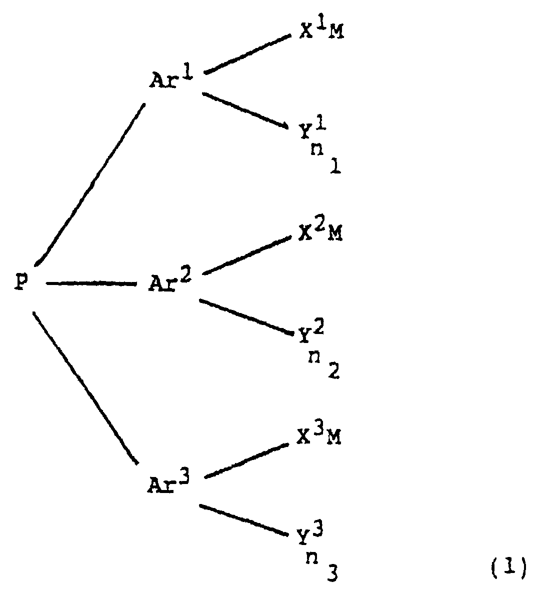 where Ar¹, Ar², Ar³ are each a phenyl or naphthyl group, Y¹, Y², Y³ are each a straight-chain or branched alkyl group having from 1 to 4 carbon atoms, an alkoxy group, a halogen atom, the OH, CN, NO₂ or R¹R²-N groups, where R¹ and R² are each a straight-chain or branched alkyl group having from 1 to 4 carbon atoms; X¹, X², X³ are each a sulfonate (SO₃ ⁻) or carboxylate (COO⁻) radical, n₁, n₂, n₃ are identical or different integers from 0 to 5. M is an alkali metal ion, one chemical equivalent of an alkaline earth metal or zinc ion, an ammonium or quaternary ammonium ion of the formula N(R³R⁴R⁵R⁶)⁺, where R³, R⁴, R⁵, R⁶ are each a straight-chain or branched alkyl group having from 1 to 4 carbon atoms.

7. The process as claimed in one or more of claims 1 to 5, wherein monophosphines used are the reaction products of dialkylphosphines or diarylphosphines with 1,2-, 1,3- or 1,4-butanesultones.

8. The process as claimed in one or more of claims 1 to 5, wherein the phosphines used are diphosphines of the formula 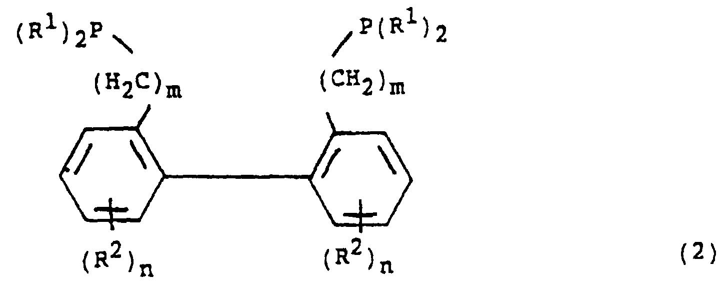 where R¹ are identical or different alkyl, cycloalkyl, phenyl, tolyl or naphthyl radicals, R² are identical or different and are hydrogen, alkyl or alkoxy radicals having from 1 to 14 carbon atoms, cycloalkyl, aryl or aroxy radicals having from 6 to 14 carbon atoms or a fused benzene ring, m are identical or different and are integers from 0 to 5 and n are likewise identical or different and are integers from 0 to 4.

9. The process as claimed in one or more of claims 1 to 8, wherein the surfactants used are cationic surfactants or amphoteric surfactants.

10. The process as claimed in claim 9, wherein the cationic surfactants are tetraalkylammonium salts.

11. The process as claimed in claim 9, wherein the amphoteric surfactants are betaines or amine oxides.

12. The process as claimed in one or more of claims 1 to 11, wherein the concentration of the surfactant in the aqueous catalyst solution lies above the critical micelle formation concentration.

13. The process as claimed in one or more of claims 1 to 12, wherein the reaction is carried out at from 120 to 140°C and from 15 to 20 MPa.

14. The process as claimed in one or more of claims 1 to 13, wherein the rhodium concentration in the aqueous catalyst solution is from 100 to 600 ppm by weight, preferably from 300 to 400 ppm by weight, based on the solution.

15. The process as claimed in one or more of claims 1 to 14, wherein at least 20 mol, preferably from 40 to 80 mol, of P(III) in the form of a phosphine are present per mole of rhodium.

16. The process as claimed in one or more of claims 1 to 15, wherein the pH of the aqueous catalyst solution is at least 3, preferably from 5 to 10 and in particular from 6 to 8.

## Revendications

1. Procédé pour la préparation d'esters d'acides di- et polyformylcarboxyliques caractérisé en ce que l'on fait réagir des esters d'acides gras plusieurs fois insaturés et des alcools monovalents de faible poids moléculaire à de 100 à 180°C et à de 5 à 35 MPa en présence d'une solution aqueuse qui contient comme catalyseurs des composés de complexes de rhodium-phosphine et en outre un agent de solubilisation (tensioactif), avec du monoxyde de carbone et de l'hydrogène.

2. Procédé selon la revendication 1, caractérisé en ce que les acides gras sont deux fois ou trois fois insaturés et contiennent de 8 à 25 atomes de carbone dans la molécule.

3. Procédé selon la revendication I ou 2, caractérisé en ce que les acides gras insaturés contiennent de 10 à 20 atomes de carbone dans la molécule.

4. Procédé selon l'une ou plusieurs quelconques des revendications 1 à 3, caractérisé en ce que les alcools monovalents de faible poids moléculaire sont saturés et contiennent de 1 à 10 atomes de carbone dans la molécule.

5. Procédé selon l'une ou plusieurs quelconques des revendications 1 à 4, caractérisé en ce que l'alcool monovalent est le méthanol.

6. Procédé selon l'une ou plusieurs quelconques des revendications 1 à 5, caractérisé en ce que la phosphine est une monophosphine de la formule générale 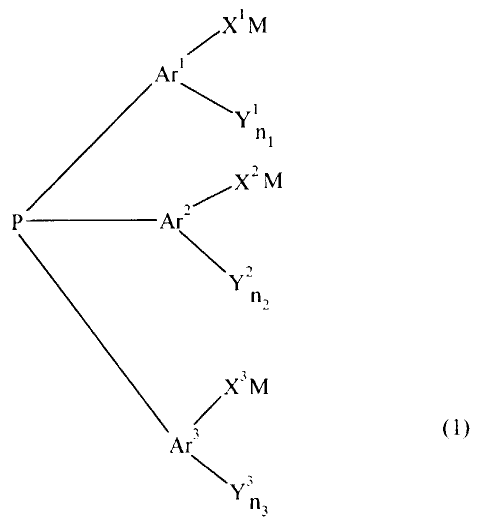 dans laquelle Ar¹, Ar², Ar³ représentent chacun un groupe phényle ou naphthyle, Y¹, Y², Y³ représentent chacun un groupe alkyle linéaire ou ramifié avec de I à 4 atomes de C, un groupe alcoxy, un atome d'halogène, les groupes OH-, CN-, NO₂- ou R¹R² N-, dans lesquels R¹ et R² représentent chacun un groupe alkyle linéaire ou ramifié avec de 1 à 4 atomes de C; X¹, X², X³ est à chaque fois un reste sulfonate (SO₃ ⁻-) et/ou carboxylate (COO⁻-), n_{1,} n₂, n₃ sont des nombres entiers identiques ou différents de 0 à 5, M est un ion de métal alcalin, un équivalent chimique d'un ion de métal alcalino-terreux ou zinc, un ion ammonium ou ammonium quaternaire de la formule générale N(R³R⁴R⁵R⁶)⁺, dans laquelle R³, R⁴, R⁵, R⁶ représentent chacun un groupe alkyle linéaire ou ramifié avec de 1 à 4 atomes de C.

7. Procédé selon l'une ou plusieurs quelconques des revendications 1 à 5, caractérisé en ce que l'on utilise comme monophosphines les produits de réaction de dialkyl- ou diarylphosphines avec des 1,2-, 1,3- ou 1,4-butanesultones.

8. Procédé selon l'une ou plusieurs quelconques des revendications 1 à 5, caractérisé en ce que l'on utilise comme phosphine des diphosphines de la formule générale 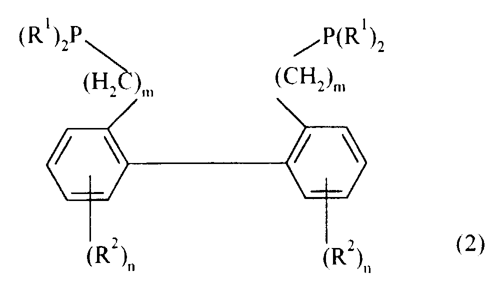 dans laquelle R¹ représente des restes alkyle, cycloalkyle, phényle, tolyle ou naphthyle identiques ou différents, R² est identique ou différent et représente l'hydrogène, des restes alkyle ou alcoxy avec de 1 à 14 atomes de carbone, des restes cycloalkyle, aryle ou aroxy avec de 6 à 14 atomes de carbone ou un noyau benzène condensé, m est identique ou différent et représente un nombre entier de 0 à 5 et n est également identique ou différent et représente un nombre entier de 0 à 4.

9. Procédé selon l'une ou plusieurs quelconques de revendications 1 à 8, caractérisé en ce que l'on utilise comme tensioactif des tensioactifs cationiques ou des tensioactifs amphotères.

10. Procédé selon la revendication 9, caractérisé en ce que les tensioactifs cationaiques sont des sels de tétraalkylammonium.

11. Procédé selon la revendication 9, caractérisé en ce que les tensioactifs amphotères sont la bétaïne ou des amino-oxydes.

12. Procédé selon l'une ou plusieurs quelconques des revendications 1 à 11, caractérisé en ce que la concentration du tensioactif dans la solution aqueuse de catalyseur est supérieure à la concentration micellaire critique.

13. Procédé selon l'une ou plusieurs quelconques des revendications 1 à 12, caractérisé en ce que la réaction a lieu à de 120 à 140°C et à de 15 à 20 MPa.

14. Procédé selon l'une ou plusieurs quelconques des revendications I à 13, caractérisé en ce que la concentration en rhodium dans la solution aqueuse de catalyseur est de 100 à 600 ppm en poids, de préférence de 300 à 400 ppm en poids, rapportées à la solution.

15. Procédé selon l'une ou plusieurs quelconques des revendications 1 à 14, caractérisé en ce qu'au moins 20 moles, de préférence de 40 à 80 moles, de P (III) sont présentes dans la forme d'une phosphine par mole de rhodium.

16. Procédé selon l'une ou plusieurs quelconques des revendications I à 15, caractérisé en ce que la valeur du pH de la solution aqueuse de catalyseur est d'au moins 3, de préférence de 5 à 10 et en particulier de 6 à 8.
